(19) Europäisches Patentamt
European Patent Office
Office européen des brevets

(11) Veröffentlichungsnummer: **0 005 518**
**B1**

(12) # EUROPÄISCHE PATENTSCHRIFT

(45) Veröffentlichungstag der Patentschrift: **16.09.81**

(21) Anmeldenummer: **79101445.9**

(22) Anmeldetag: **11.05.79**

(51) Int. Cl.³: **C 07 D 213/20,** C 07 D 213/24, C 07 D 213/26, C 07 D 215/10, C 07 D 215/12, G 01 N 33/52

(54) **Bis-(2,4-dinitrophenyl)-methyl-pyridinium-Verbindungen, Verfahren zu ihrer Herstellung und ihre Verwendung als pH-Indikatoren.**

(30) Priorität: **17.05.78 DE 2821501**

(43) Veröffentlichungstag der Anmeldung: **28.11.79 Patentblatt 79/24**

(45) Bekanntmachung des Hinweises auf die Patenterteilung: **16.09.81 Patentblatt 81/37**

(84) Benannte Vertragsstaaten: **AT BE CH DE FR GB IT LU NL SE**

(56) Entgegenhaltungen:
**US-A-2 632 761**

**CHEMICAL ABSTRACTS, Band 33, 10-05-1939 Spalte: 42467-8, Seite 1939 COLUMBUS OHIO (US)**

**CHEMICAL ABSTRACTS, Band 44, 10-11-1950 Spalte: 9961 g, Seite 1950 COLUMBUS OHIO (US)**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Rothe, Anselm, Dr., Im Schwanklingen 20, D-6943 Birkenau (DE)**
Erfinder: **Guthlein, Werner Dr., Im Senntelch 31, D-6800 Mannheim 24 (DE)**
Erfinder: **Rittersdorf, Walter, Dr., Kasseler-Strasse 6, D-6800 Mannheim 31 (DE)**
Erfinder: **Werner, Wolfgang, Dr., Meissener Weg 39, D-6800 Mannheim 42 (DE)**

(56) Entgegenhaltungen:
**CHEMICAL ABSTRACTS, Band 84, No. 14, 5-04-1976 Zusammenfassung: 91612z, Seite 82 COLUMBUS OHIO (US)**

**EP 0 005 518 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Bis-(2,4-dinitrophenyl)-methyl-pyridinium-Verbindungen, Verfahren zu ihrer Herstellung und ihre Verwendung als pH-Indikatoren

Die Remissionsphotometrie hat sich in der klinischen Chemie speziell wegen der einfachen und unkomplizierten Auswertung von Teststreifen und der damit verbundenen Einsparung von Kosten durchgesetzt.

Die Messungen sollen mit möglichst preiswerten Geräten durchgeführt werden, die im Gegensatz zu Spektral- und Linienphotometern in einem breiten Spektralbereich arbeiten. Ein solches Gerät ist beispielsweise in der DE-PS 20 56 232 beschrieben.

Zur Gewährleistung einer ausreichenden Messempfindlichkeit müssen Teststreifen, die mit diesen Geräten gemessen werden, Indikatoren enthalten, die keine steilen Absorptionspeaks, sondern breite Absorptionsbanden aufweisen.

Ein grosser Anteil der Absorptionsbande soll zur Emissionscharakteristik der in solchen Geräten zweckmässigerweise verwendeten Glimmlampen passen und somit oberhalb 600 nm liegen. Zudem muss von den Indikatoren gefordert werden, dass die Molekülform, die nicht zur Messung herangezogen wird, nur in einem Bereich absorbiert, den das Remissionsphotometer nicht registriert, um einen möglichst grossen Farbenunterschied und damit ein deutliches Messignal durch die Reaktion zu erzeugen. Das bedeutet in der Regel, dass diese Form ein farbloses bis gelbes Aussehen besitzen muss.

Ein solcher Teststreifen ist z.B. für die schnelle und quantitative Glucosebestimmung aus Körperflüssigkeiten wie Blut, Serum etc. in der DE-PS 22 64 438 beschrieben.

Entsprechend den oben erwähnten Anforderungen an Indikatoren zur Messung an preiswerten Remissionsphotometern enthält dieser Teststreifen Derivate des Benzidins. Sie sind in ihrer reduzierten Form farblos-gelb, absorbieren somit in einem sehr niederwelligen Bereich. Die Oxidationsprodukte hingegen absorbieren über einen breiten Wellenlängenbereich bis ins nahe IR.

Zur Bestimmung des Harnstoffgehalts in Blut war bisher noch kein Teststreifen bekannt, der einfache Handhabung mit kurzer Reaktionszeit und einer präzisen remissionsphotometrischen Auswertung verband. Dabei ist es besonders in Notfällen, z.B. bei einem urämischen Koma, für die einzuleitenden therapeutischen Massnahmen unerlässlich, in kürzester Zeit ein möglichst genaues Analysenergebnis zu erhalten. Aber auch im Routinebetrieb des klinischen Labors wäre mit der Einführung eines remissionsphotometrisch auswertbaren Harnstoff-Schnelltestes ein deutlicher Fortschritt zu erzielen, da nunmehr langwieriges Pipettieren, Umgang mit zum Teil wenig haltbaren und ätzenden Reagenzien und eine lange Reaktionszeit wegfielen.

Die Entwicklung eines solchen Harnstoff-Teststreifens scheiterte bisher am Mangel an geeigneten Indikatoren. Im Gegensatz zum oben beschriebenen Glucose-Teststreifen mit einem Redox-Indikator werden in der klinischen Chemie zum Nachweis von Harnstoff als Farbindikatoren in der Reaktionskette pH-Indikatoren zum Nachweis des gebildeten Ammoniaks verwendet.

Bisher waren keine pH-Indikatoren bekannt, die neben leichter Zugänglichkeit die obengenannten spektralen Anforderungen erfüllten und zugleich ausreichende Löslichkeit und einen passenden pK-Wert aufwiesen.

Zwei bekannte Verbindungen – Hexanitrotriphenylmethane und 2-Bis(2,4-dinitrophenyl)-essigsäureethylester – besitzen zwar eine brauchbare Absorption, andererseits aber eine zu geringe Löslichkeit bzw. einen zu hohen pK-Wert.

Andere bekannte pH-Indikatoren sind nicht verwertbar, da entweder sowohl die saure als auch die basische Form über einen breiten Wellenlängenbereich absorbieren und deshalb remissionsphotometrisch nicht scharf voneinander getrennt werden, oder die gefärbte Form nur in einem mehr oder minder steilen Peak absorbiert, so dass eine Messung mit einfachen Geräten, die nur einen breiten Wellenlängenbereich erfassen, nicht möglich ist.

Die Aufgabe der vorliegenden Erfindung war es deshalb, pH-Indikatoren, die in ihrer sauren Form ungefärbt und in ihrer basischen Form stark gefärbt sind, zu schaffen, wobei sich die Färbung durch eine breite Absorptionsbande auszeichnen muss. Mindestens eine Form dieser Indikatoren muss eine ausreichende Löslichkeit aufweisen, so dass sie zu Teststreifen verarbeitet werden können. Es wird ausserdem ein ausreichend niedriger pK-Wert (z.B. 5–10,5) verlangt, der die Einstellung eines für einen Harnstoff-Teststreifen vernünftigen, d.h. der Nachweis von Ammoniak erlaubenden pH-Bereichs ermöglicht.

In den Bis-(2,4-dinitrophenyl)-methyl-pyridinium-Verbindungen der allgemeinen Formel I wurden Verbindungen gefunden, die diese Anforderungen erfüllen.

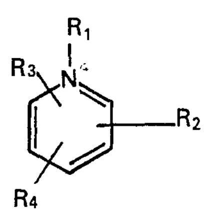

X (I)

wobei eine der Gruppen $R_1$ und $R_2$ eine Bis-(2,4-dinitrophenyl)-methylgruppe und die andere eine niedere Alkylgruppe, insbesondere Methyl- oder t.Butylgruppe darstellt oder für den Fall, dass $R_1$ die Bis-(2,4-dinitrophenyl)-methylgruppe darstellt, $R_2$ Wasserstoff bzw. eine Trifluormethylgruppe bedeuten,
und $R_3$ und $R_4$ Wasserstoff oder zusammen mit den Kohlenstoffatomen, an die sie gebunden sind, einen Benzolring darstellen,
und $X^\ominus$ ein geeignetes Säureanion darstellt bzw. die Verbindung als Betain vorliegt.

Die erfindungsgemässen neuen Verbindungen können hergestellt werden, indem man
a) eine Verbindung der Formel I'

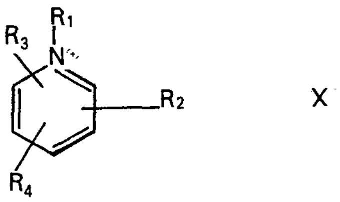

(I'),

in der eine der Gruppen $R_1$ und $R_2$ eine Diphenylmethylgruppe darstellt und die anderen Gruppen die gleiche Bedeutung haben wie in der Formel I, nitriert.
b) für den Fall, dass $R_1$ die Bis-(2,4-dinitrophenyl)-methylgruppe darstellt eine Verbindung der Formel II

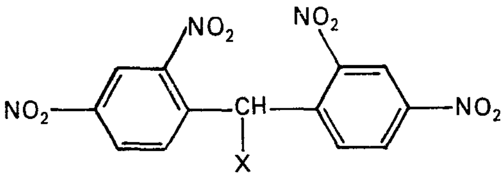

(II),

in der X ein Cl, Br oder J darstellt,
mit einer Verbindung der Formel III

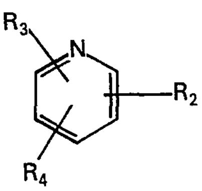

(III),

in der $R_2$ Wasserstoff oder eine niedere Alkylgruppe darstellt und $R_3$ und $R_4$ die obige Bedeutung haben,
umsetzt,
worauf die Gruppe $X^\ominus$ in an sich bekannter Weise durch eine andere Gruppe $X^{\ominus\prime}$ ersetzt oder eine Alkylgruppe $R_1$ eingeführt werden kann.

Die Tetranitrierung von Verbindungen der Formel I' erfordert relativ energische Bedingungen und wird deshalb bevorzugt mit einem Gemisch von rauchender Salpetersäure und Oleum bei Temperaturen von 20–100°C durchgeführt. Die Gruppe $X^\ominus$ wird dabei entweder vorher oder durch den Schwefelsäureüberschuss im Reaktionsmedium in die Sulfatgruppe überführt.

Die Umsetzung der Verbindungen der Formeln II und III erfolgt vorzugsweise in einem Überschuss der Verbindung III als Lösungsmittel, es kann jedoch auch ein anderes inertes Lösungsmittel wie aromatische Kohlenwasserstoffe, Ether, Dioxan, Aceton etc. verwendet werden. Die Verbindung II kann entweder aus dem entsprechenden, bekannten Carbinol durch Umsetzen mit der Halogenwasserstoffsäure oder aus der Methylenverbindung durch Umsetzung mit dem entsprechenden elementaren Halogen in einem inerten Lösungsmittel hergestellt werden. Diese Verfahren können auch zur Herstellung der Ausgangsverbindungen der Formel I' verwendet werden, soweit diese noch nicht literaturbekannt ist.

Besonders bevorzugt ist es, die der Verbindung II entsprechenden Methylenverbindungen der folgenden Formel II'

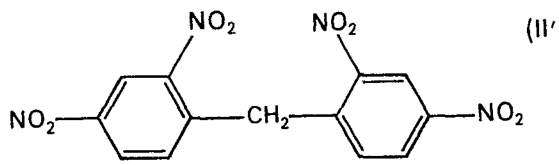

(II'),

die durch Nitrieren von Diphenylmethan nach K. Matsumara, J. Am. Chem. Soc. 51, 817 (1929) hergestellt werden kann, in einem Überschuss des Pyridins III zu lösen und die Verbindung II in situ durch langsames Zufügen von molaren Mengen Brom oder Jod zu erzeugen. Gegebenenfalls kann ein inertes Lösungsmittel wie Benzol, Ether etc. zugefügt werden. Die gewünschten Verbindungen der Formel I fallen dabei in schwerlöslicher Form als Halogenide aus.

Die Einführung einer Alkylgruppe $R_1$ in die Ausgangsverbindung I' erfolgt in an sich bekannter Weise mit einem Alkylierungsmittel beispielsweise Alkylhalogenid, Dialkylsulfat oder Alkylsulfonsäureester in inerten Lösungsmitteln wie Toluol, Ether, Dioxan, Aceton etc.

Der Austausch einer Gruppe $X^\ominus$ gegen eine andere erfolgt durch Umsetzung mit einem entsprechenden Überschuss der entsprechenden Säure HX oder vorzugsweise eines ihrer Salze, dessen Kation mit der zu entfernenden Gruppe $X^\ominus$ ein schwerlösliches, leicht abtrennbares Salz bildet. Ferner lässt sich durch Zugabe einer Base, wie wässriges Ammoniak oder Alkali das Betain herstellen, das gewünschtenfalls durch Zugabe einer anderen Säure wieder in ein neues quaternäres Salz überführt werden kann.

Bevorzugte Anwendung finden die erfindungsgemässen pH-Indikatoren in Harnstoff-Testen. Wie im folgenden Beispiel 7 gezeigt wird, ist mit den erfindungsgemässen Verbindungen auch ein quantitativer Harnstoff-Test herstellbar, der 66,5% des Messbereiches des Remissionsphotometers umfasst. Bei der Verwendung eines herkömmlichen pH-Indikators (z.B. Bromphenolblau) wird nur ein visuell ablesbarer semiquantitativer Test erhalten (siehe Beispiel 8), da er nur ca. 23% des Messbereiches überstreicht. Selbstverständlich sind sie aber auch als pH-Indikatoren selbst, gegebenfalls auch zusammen mit anderen pH-Indikatoren in Mischindikatoren für grössere pH-Bereiche einsetzbar.

Die Erfindung wird in den folgenden Beispielen näher erläutert:

Beispiel 1
1-[Bis-(2,4-dinitrophenyl)methyl]-4-tert.-butylpyridinium-halogenide
a) Bromid

Man suspendiert 26,4 g (0,076 mol) Bis-(2,,4-dinitrophenyl)-methan in 125 ml = 114,38 g (0,976

mol) tert. Butylpyridin und tropft bei 0–5°C 12,4 g = 4 ml (0,155 mol) trockenes Brom langsam unter Rühren zu. Danach rührt man 6 d bei Raumtemperatur, saugt das abgeschiedene Reaktionsprodukt scharf ab, wäscht mit 10 ml tert. Butylpyridin, rührt den Filterkuchen mit 100 ml 10-proz. Bromwasserstoffsäure an, kühlt durch Einstellen in ein Eisbad und saugt abermals scharf ab. Den Filterrückstand löst man unter Erwärmen in 200 ml Methanol, saugt eventuell vorhandene unlösliche Anteile ab und fällt das Bromid durch Zugabe von 50 ml Ether. Nach Ausrühren mit 50 ml Aceton erhält man 12,42 g = 31,5% reines 1-[Bis-(2,4-dinitrophenyl)methyl]-4-tert.-butylpyridinium-bromid; Schmp.: 204–206°C. Aus dem eingeengten Filtrat und aus der aufgearbeiteten Mutterlauge (Ansäuern mit überschüssiger 10-proz. Bromwasserstoffsäure, Absaugen des gefällten Materials) resultieren nach Umkristallisieren aus Methanol-Ether und Anrühren mit Aceton weitere 4,1 g = 9,6% der obengenannten Verbindung.

DC: Fertigplatte, Kieselgel 60 F 254 (Merck)
Laufmittel:
Isopropanol-Essigester-$H_2O$ 5:3:2,
RF-Wert = 0,6
Toluol-Aceton 3:7, RF-Wert = 0,6
(Beide Laufmittel in Gegenwart von gasf. Ammoniak)
Detektion: Ammoniak (Gas)

b) Iodid

In analoger Weise gewinnt man 1-[Bis-(2,4-dinitrophenyl)-methyl]-4-tert.-butylpyridinium-iodid, Schmp.: 217°C, Ausbeute: 25,6%. (Bei der Darstellung verwendet man anstelle von elementarem Iod eine Lösung desselben in tert. Butylpyridin.)

c) Chlorid

In gleicher Weise gelingt auch die Herstellung von 1-[Bis-(2,4-dinitrophenyl)methyl]-4-tert.-butylpyridinium-chlorid, Schmp. 172–173°C, Ausbeute: 31,6%. Anstelle von elementarem Chlor verwendet man eine Lösung von Chlor in Eisessig, sonst verfährt man wie unter a) und b) angegeben.

Sowohl Iodid wie Chlorid verhalten sich im DC wie das Bromid, wenn die dort angegebenen Laufmittel verwendet werden.

Beispiel 2

1-[Bis-(2,4-dinitrophenyl)methyl]pyridinium-bromid

Unter Verwendung von Pyridin anstelle von 4-tert.-Butylpyridin wird wie in Beispiel 1 verfahren. Man erhält dünnschichtchromatographisch einheitliches 1-[Bis-(2,4-dinitrophenyl)methyl]-pyridinium-bromid, MG: 506,24, Schmp.: 199–200°C, Ausbeute: 25,6%.

Aus der methanolischen Lösung des Bromids fällt mit 35-proz. Tetrafluorborsäure das Tetrafluoroborat, MG: 513,26, Schmp.: 190–191°C.

DC: Fertigplatte, Kieselgel 60 F 254 (Merck)
Laufmittel: Aceton, RF-Wert = 0,4
Detektion: UV, Ammoniak (Gas)

Beispiel 3

1-[Bis-(2,4-dinitrophenyl)methyl]chinolinium-bromid

Man verwendet anstelle von 4-tert.-Butylpyridin Chinolin. Die Umsetzung und Aufarbeitung erfolgt gemäss Beispiel 1. Durch fraktionierte Kristallisation aus Methanol-Ether und Aceton-Ether erhält man chromatographisch einheitliches 1-[Bis-(2,4-dinitrophenyl)-methyl]-chinolinium-bromid, Schmp. 186–188°C, Ausbeute: 25,2%.

DC: Fertigplatte, Kieselgel 60 F 254 (Merck)
Laufmittel: Aceton, RF-Wert = 0,6
Detektion: Ammoniak (Gas)

Beispiel 4

1-[Bis-(2,4-dinitrophenyl)methyl]pyridinium-tetrafluoroborat

Man löst 16,3 g (0,05 mol) Diphenyl-methyl-pyridiniumbromid (erhalten aus Diphenylbrommethan durch Erhitzen mit überschüssigem Pyridin, (30 sec./120°C), Einengen, Anrühren mit Ether, Schmp: 216–219°C) in 30 ml konz. Schwefelsäure, leitet 1 h $CO_2$ durch die Lösung, erwärmt auf 80°C, kühlt auf Raumtemperatur ab und tropft die schwefelsaure Lösung innerhalb 15 min. in ein Nitriergemisch aus 30 ml konz. Schwefelsäure und 8,65 g rauchender Salpetersäure und hält dabei die Temperatur unter 50°C. Anschliessend tropft man ein Gemisch von 11,4 ml Salpetersäure, (D = 1,52) und 42,5 ml Oleum (15% $SO_3$-Gehalt) zu. Danach erwärmt man 3 h auf 70°C und giesst nach Abkühlen auf 600 ml Eis. Die gelbe Fällung wird in Aceton-Isopropanol gelöst und durch Zugabe von Ether ausgefällt. Das Rohprodukt, das ausser der gewünschten Verbindung noch mehrere Nebenprodukte enthält, wird an einer Kieselgelsäule mit Aceton als Lösungsmittel chromatographisch getrennt. Man erhält 5,6 g chromatographisch einheitliches, amorphes Hydrogensulfat, das in Methanol mit wässriger 38-proz. Borfluorwasserstoffsäure 4,8 g = 20,7% 1-[Bis-(2,4-dinitrophenyl)-methyl]pyridinium-tetrafluoroborat, Schmp.: 187–191°C ergibt.

Beispiel 5

(Diphenylmethyl)-1-methylpyridinium-iodide

Man suspendiert 0,1 mol Diphenyl-pyridylmethan in 200 ml Toluol, gibt 51,25 g = 45 ml (0,36 mol) Methyliodid zu und erhitzt 2 h unter Rückfluss. Man kühlt auf Raumtemperatur, gibt zu der Reaktionsmischung 50 ml Ether, saugt die abgeschiedenen Kristalle ab, wäscht gut mit Ether und trocknet über Diphosphorpentoxid. Je nach Substitution der Ausgangsverbindung werden die folgenden Produkte erhalten:

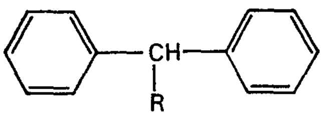

MG: 387.2

| R | | Schmelz-punkt | Ausbeute |
|---|---|---|---|
| 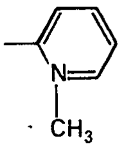 | J | 228 °C Zers.) | 98,1% |
| 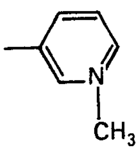 | J | 160 °C (Zers.) | 91,6% |
| 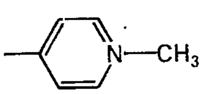 | J | 152–155 °C (Zers.) | 96,5% |

(Diphenylmethyl)-1-methylpyridinium-nitrate

Man löst 0,1 mol der vorstehenden (Diphenylmethyl)-1-methylpyridinium-iodide in 500 ml Methanol, gibt eine Lösung von 0,1 mol Silbernitrat in einer Mischung von 400 ml Methanol und100 ml Wasser zu und rührt eine Stunde bei Raumtemepratur, danach saugt man das gebildete Silberiodid ab und engt das Filtrat am Rotationsdampfer ein. Den Destillationsrückstand löst man in wenig Methanol und fällt das Nitrat durch Zugabe von Ether. Nach Absaugen, Waschen mit Ether und Trocknen über Diphosphorpentoxid erhält man die reinen Nitrate in Form farbloser Kristalle.

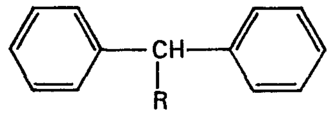

MG: 322.3

| R | | Schmelz-punkt | Ausbeute |
|---|---|---|---|
| 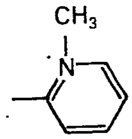 | $NO_3$ | 171 °C | 93,8% |
| 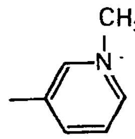 | $NO_3$ | 138 °C | 88,4% |
| 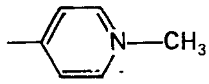 | $NO_3$ | 176–178 °C | 96,3% |

[Bis-(2,4-dinitrophenyl)methyl]-1-methylpyridinium-hydrogensulfate

In einem 500 ml Dreihalskolben mit Rührer, Kühler, Thermometer und Tropftrichter gibt man 17,8 ml = 27,2 g = 0,431 mol Salpetersäure (D = 1,52) in 90 g = 50 ml = 0,98 mol konz. Schwefelsäure (D = 1,84) und fügt unter Rühren 16,1 g (0,05 mol) eines der vorstehenden (Diphenylmethyl)-1-methylpyridinium-nitrate so zu, dass die Temperatur der Reaktionsmischung 50 °C nicht überschreitet. Danach erwärmt man langsam auf 70 °C und tropft bei dieser Temperatur eine Mischung von 60 ml Oleum (15% $SO_3$-Gehalt) und 30 ml = 47,5 g = 0,75 mol Salpetersäure (D = 1,52) zu, erwärmt eine Stunde auf 90 °C und giesst anschliessend auf 500 ml Eis. Das erhaltene Rohprodukt wird in wenig Methanol unter Erwärmen gelöst, mit Aktivkohle behandelt, abgesaugt und das Hydrogensulfat durch Zugabe von Ether gefällt. Durch nochmaliges Umlösen aus Aceton-Ether erhält man die chromatographisch einheitlichen amorphen Hydroxysulfate der Tetranitroverbindungen.

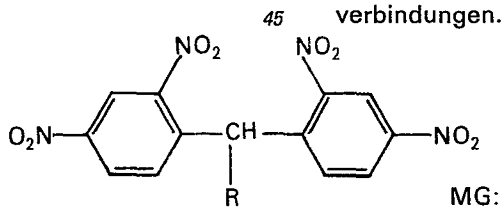

MG: 537.3.

| R | | Schmp. | Ausbeute | DC* Laufmittel | RF |
|---|---|---|---|---|---|
| 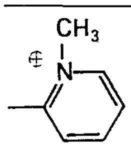 | $HSO_4^{\ominus}$ | amorph Sintern ab 70 °C | 31,5% | XM Xylol-Methyl-ethylketon 1:1 | 0,21 |
| 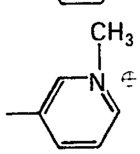 | $HSO_4$ | amorph Sintern ab 120 °C Zers. | 25,6% | DEW 211 n-Butanol: Eisessig-Wasser 2:1:1 | 0,19 |

| R | Schmp. | Ausbeute | DC* Laufmittel | RF |
|---|---|---|---|---|
| 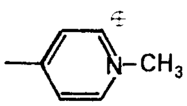 $HSO_4$ | amorph Sintern ab 70°C | 32,1% | Isopropanol Essigsäure-n butylester: Wasser-Ammoniak 50:30:15:8 | 0,45 |

*DC-Fertigplatte, Dieselgel 60 F 254 (Merck)

Bis-(2,4-dinitrophenyl)-(1-methyl-x-pyridinio)methanide

Zu ihrer Darstellung löst man die vorstehenden Hydrogensulfate in Wasser und fügt Ammoniak oder verdünnten Alkali bis pH 9–10,5 zu. Die tiefblau bis violett gefärbten Pyridiniobetaine werden abgesaugt, mit Wasser, Petrolether-Ligroin 1:1 gewaschen und über Diphosphorpentoxid getrocknet. Beim Erhitzen zersetzen sie sich ohne zu schmelzen.

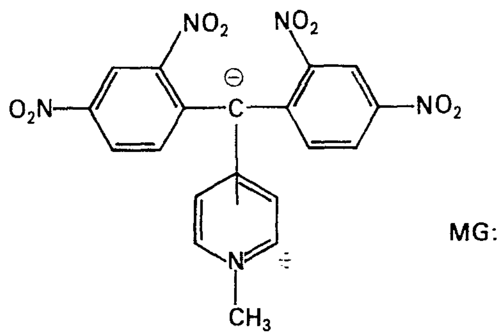

MG: 438.5

| Stellung x | Zersetzungsprodukt | Ausbeute | pK-Wert | Absorption [nm] |
|---|---|---|---|---|
| 2 | >225°C | 90,2% | 9 | 400–800 |
| 3 | >238°C | 93,5% | 10,5 | 400–900 |
| 4 | >233°C | 95,1% | 6,5 | 400–810 |

Beispiel 6

Bis-(2,4-dinitrophenyl)-(4-tert.-Butyl-1-pyridinio)-methanid

Man löst 40 g (0,071 mol) 1-[Bis-(2,4-dinitrophenyl)methyl]-(4-tert.-butyl-1-pyridinium)bromid oder -Tetrafluoroborat (hergestellt aus der methanolischen Lösung des rohen Bromids durch Zugabe von 38-proz. Tetrafluoroborsäure, Schmp.: 204–205°C) in 300 ml Dimethylformamid unter Zugabe von 2 ml 48-proz. Bromwasserstoffsäure und stellt unter starkem Rühren und Kontrolle mit einer Glaselektrode durch Zutropfen von 950 ml 5-proz. ethanolischer Kalilauge auf pH 10. Danach giesst man das abgeschiedene 1-[Bis-(2,4-dinitrophenyl)-4-tert.-Butyl-1-pyridino]-methanid ab, wäscht mit 3 Liter Wasser und trocknet das Betain über Kieselgel und Diphosphorpentoxid. Man erhält 33,5 g = 97,8% metallisch glänzende Kristalle der Titelverbindung. Die Verbindung zersetzt sich bei 225°C, ohne zu schmelzen.

DC: Fertigplatte, Kieselgel 60 F 254 (Merck)
Laufmittel: Isopropanol: Essigsäure-n-butylester-Wasser 5:3:2
RF-Wert = 0,63

Man kann zur Darstellung der Pyridiniumbetaine auch so verfahren, dass man die DMF-Lösung des betreffenden Salzes in eine Mischung aus DMF-Ammoniak einträgt, auf pH 9–10 einstellt, anschliessend durch Zugabe der vierfachen Menge Wasser das Betain zur Abscheidung bringt und wie zuvor angegeben weiterbehandelt.

In gleicher Weise werden die folgenden Betaine der folgenden allgemeinen Formel hergestellt

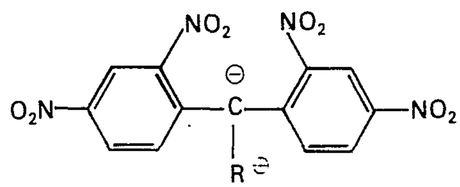

| $R^{\oplus}$ | Zersetzungspunkt | Ausbeute | pK-Wert | Absorption (nm) |
|---|---|---|---|---|
| 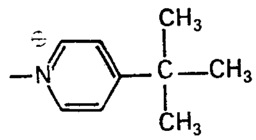 MG: 481.4 | >225 °C | 97,8% | 8,5 | 400–850 Das Absorptionsspektrum ist in Fig. 2 abgebildet |
| 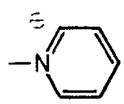 MG: 425.3 | >250 °C | 96,5% | 8,5 | 400–800 |
| 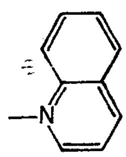 MG: 475.3 | >210 °C | 95,6% | 8 | 400–850 |
| 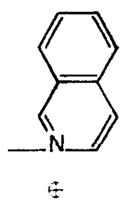 MG: 475.3 | 230 °C | 96,6% | 7 | 400–850 |

Beispiel 7

Quantitativer Nachweis von Harnstoff in Serum

a) Ureasepapier

Filterpapier wird mit einer Lösung folgender Zusammensetzung getränkt, getrocknet und in 6 mm breite Bänder zerschnitten.

| | |
|---|---|
| Urease (5 U/mg) | 6 g |
| Dithioerythrit | 0,1 g |
| 0,3 m TRIS.HCl-Puffer pH 8,5 | 100 ml |

b) Indikatorpapier

Filterpapier wird mit einer Lösung folgender Zusammensetzung getränkt, getrocknet und ebenfalls in 6 mm breite Streifen geschnitten.

| | |
|---|---|
| N-(Bis-(2,4-dinitrophenyl)-methyl)-4-t.butyl-pyridiniumchlorid | 0,39 g |
| Ethylenglykolmonomethylether | 42 ml |
| 0,25 m Na-Malonat-Puffer pH 2,8 | 48 ml |

c) Abstandshalter

Mit Silikonharz hydrophobiertes Siebdruckgewebe von ca. 100 µ Fadendicke und mit einer offenen Siebfläche von ca. 35% wird in 25–40 mm breite Bänder geschnitten.

d) Abdecknetz

Hydrophiles Nylonnetz von ca. 60 µ Dicke, Fadenstärke 40 µ, freie Lochfläche ca. 65% wird in 15 mm breite Bänder geschnitten.

e) Folie

Als Trägerfolie und Handgriff wird ein 6–10 mm breites, ca. 0,2–0,3 mm dickes Band aus schmelzkleberbeschichteter Polyesterfolie verwendet.

Herstellung der Teststreifen

Ureasepapier (3), Indikatorpapier (1) und Abstandshalter (2) werden zusammen mit einem das Ureasepapier abdeckenden Netz (4) wie in

Fig. 1 gezeigt auf das Ende der mit Schmelzkleber beschichteten 6–10 cm breiten Folie (5) gesiegelt und das entstehende Band in 6 mm breite Streifen geschnitten, so dass 6×6 mm-Testbezirke an einem 6–10 cm langen Handgriff entstehen.

Ein solcher Streifen wird auf das Abdecknetz mit 10 µl Serum betropft und mit einem Klebeetikett verschlossen. Nach einer Reaktionszeit von 7 Minuten werden Ureasepapier und Abdecknetz zusammen mit der Abstandsschicht entfernt. Die Verfärbung der Indikatorschicht wird von oben mit einem Remissionsphotometer vermessen. In Abhängigkeit von der Harnstoffkonzentration werden folgende Messwerte erhalten:

| mg Harnstoff/100 ml Serum | Messignal (Skalenteile) ±1 s Mittelwert aus 10 Werten |
|---|---|
| 20 | 12,9±0,75 |
| 40 | 27,5±1,5 |
| 60 | 46,1±1,9 |
| 80 | 61,5±1,4 |
| 100 | 69,0±0,4 |
| 150 | 77,2±0,5 |
| 200 | 79,4±0,5 |

Beispiel 8

Semiquantitativer Nachweis von Harnstoff in Blut

a) Ureasepapier
siehe Beispiel 7

b) Indikatorpapier

Filterpapier wird mit einer Lösung folgender Zusammensetzung getränkt und bei 70°C getrocknet

| | |
|---|---|
| Bromphenolblau | 0,1 g |
| Ethylenglykol-monomethylether | 9 ml |
| Weinsäure | 0,4 g |
| Wasser | 21 ml |

c) Abstandshalter

Mit Silikonharz hydrophob ausgerüstetes Polyamidvlies, Dicke ca. 80 µ

d) Abdecknetz
siehe Beispiel 7

Verarbeitung siehe Beispiel 7.

Zur Harnstoffbestimmung in Vollblut wird mit einem Tropfen Blut betropft. Nach einer Reaktionszeit von 7 Minuten entstehen je nach Harnstoffgehalt visuell gut unterscheidbare Reaktionsfarben:

| | |
|---|---|
| 20 mg Harnstoff/100 ml Blut | gelb |
| 40 mg Harnstoff/100 ml Blut | grünlich-gelb |
| 60 mg Harnstoff/100 ml Blut | gelbgrün |
| 80 mg Harnstoff/100 ml Blut | grün |
| 100 mg Harnstoff/100 ml Blut | blaugrün |
| 150 mg Harnstoff/100 ml Blut | grünlich-blau |
| 200 mg Harnstoff/100 ml Blut | blau |

**Patentansprüche** für Vertragsstaaten: BE, CH, DE, FR, GB, IT, LU, NL, SE

1. Bis-(2,4-dinitrophenyl)-methyl-pyridinium-Verbindungen der allgemeinen Formel I

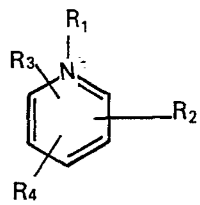 X (I)

wobei eine der Gruppen $R_1$ und $R_2$ eine Bis-(2,4-dinitrophenyl)-methylgruppe und die andere eine niedere Alkylgruppe, insbesondere Methyl- oder t.Butylgruppe darstellt oder für den Fall, dass $R_1$ die Bis-(2,4-dinitrophenyl)-methylgruppe darstellt, $R_2$ Wasserstoff bzw. eine Trifluormethylgruppe bedeuten
und $R_3$ und $R_4$ Wasserstoff oder zusammen mit den Kohlenstoffatomen, an die sie gebunden sind, einen Benzolring darstellen,
und $X^{\ominus}$ ein geeignetes Säureanion darstellt bzw. die Verbindung als Betain vorliegt.

2. Verfahren zur Herstellung von Bis-(2,4-dinitrophenyl)-methyl-pyridinium-Verbindungen gemäss Anspruch 1, dadurch gekennzeichnet, dass man
a) eine Verbindung der Formel I′

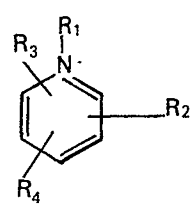 X (I′),

in der eine der Gruppen $R_1$ und $R_2$ eine Diphenylmethylgruppe darstellt und die anderen Gruppen die gleiche Bedeutung haben wie in der Formel I, nitriert oder
b) für den Fall, dass $R_1$ die Bis-(2,4-dinitrophenyl)-methylgruppe darstellt eine Verbindung der Formel II

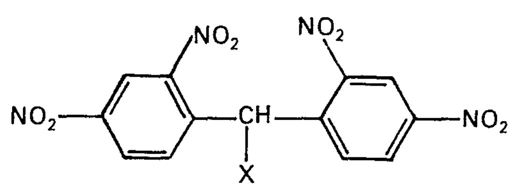 (II),

in der X ein Cl, Br oder J darstellt,
mit einer Verbindung der Formel III

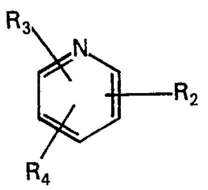

(III),

in der $R_2$ Wasserstoff oder eine niedere Alkylgruppe darstellt und $R_3$ und $R_4$ die obige Bedeutung haben,
umsetzt,
worauf die Gruppe $X^{\ominus}$ in an sich bekannter Weise durch eine andere Gruppe $X^{\ominus\prime}$ ersetzt oder eine Alkylgruppe $R_1$ eingeführt werden kann.

3. Verwendung von Bis-(2,4-dinitrophenyl)-methyl-pyridinium-Verbindungen gemäss Anspruch 1 als pH-Indikatoren.

4. Verwendung gemäss Anspruch 3 zur Bestimmung von Ammoniak in Harnstofftesten.

5. PH-Bestimmungsmittel gekennzeichnet durch einen Gehalt an Verbindungen gemäss Anspruch 1.

**Patentanspruch** für Vertragsstaat AT

Verfahren zur Herstellung von Bis-(2,4-dinitrophenyl)-methyl-pyridinium-Verbindungen der allgemeinen Formel I

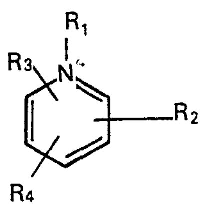

X (I)

wobei eine der Gruppen $R_1$ und $R_2$ eine Bis-(2,4-dinitrophenyl)-methylgruppe und die andere eine niedere Alkylgruppe, insbesondere Methyl- oder t.Butylgruppe darstellt oder für den Fall, das $R_1$ die Bis-(2,4-dinitrophenyl)-methylgruppe darstellt, $R_2$ Wasserstoff bzw. eine Trifluormethylgruppe bedeuten
und $R_3$ und $R_4$ Wasserstoff oder zusammen mit den Kohlenstoffatomen, an die sie gebunden sind, einen Benzolring darstellen,
und $X^{\ominus}$ ein geeignetes Säureanion darstellt bzw. die Verbindung als Betain vorliegt,
dadurch gekennzeichnet, dass man
a) eine Verbindung der Formel I'

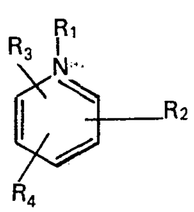

X (I'),

in der eine der Gruppen $R_1$ und $R_2$ eine Diphenylmethylgruppe darstellt und die anderen Gruppen die gleiche Bedeutung haben wie in der Formel I, nitriert.
b) für den Fall, dass $R_1$ die Bis-(2,4-dinitrophenyl)-methylgruppe darstellt eine Verbindung der Formel II

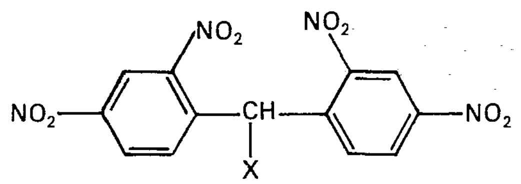

(II),

in der X ein Cl, Br oder J darstellt,
mit einer Verbindung der Formel III

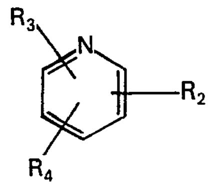

(III),

in der $R_2$ Wasserstoff oder eine niedere Alkylgruppe darstellt und $R_3$ und $R_4$ die obige Bedeutung haben,
umsetzt,
worauf die Gruppe $X^{\ominus}$ in an sich bekannter Weise durch eine andere Gruppe $X^{\ominus\prime}$ ersetzt oder eine Alkylgruppe $R_1$ eingeführt werden kann.

**Claims** for the Contracting States: BE, CH, DE, FR, GB, IT, LU, NL, SE

1. Bis-(2,4-dinitrophenyl)-methylpyridinium compounds of the general formula I

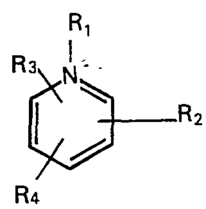

X (I)

wherein one of the groups $R_1$ and $R_2$ represents a bis-(2,4-dinitrophenyl)-methyl group and the other a lower alkyl radical, especially methyl or t.-butyl group, or, for the case in which $R_1$ represents the bis-(2,4-dinitrophenyl)-methyl group, $R_2$ signifies hydrogen atom or a trifluoromethyl group and $R_3$ and $R_4$ represent hydrogen or, together with the carbon atoms to which they are attached, a benzene ring, and $X^{\ominus}$ represents a suitable acid anion or the compound is present as betaine.

2. Process for the preparation of bis-(2,4-dinitrophenyl)-methyl-pyridinium compounds according to claim 1, characterised in that one
a) nitrates a compound of the formula I'

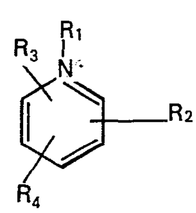

X (I'),

in which one of the groups $R_1$ and $R_2$ represents a diphenylmethyl group and the other groups have the same meaning as in formula I, or
b) for the case in which $R_1$ represents a bis-(2,4-dinitrophenyl)-methyl group, reacts a compound of the formula II

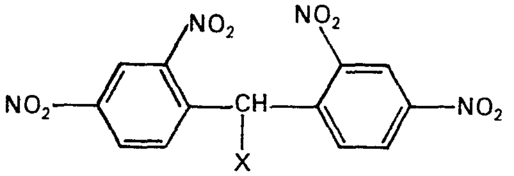

(II),

in which X represents a Cl, Br or I, with a compound of the formula III

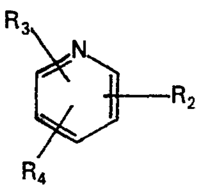

(III),

in which $R_2$ represents hydrogen or a lower alkyl group and $R_3$ and $R_4$ have the above meaning, whereafter the group $X^{\ominus}$ can be replaced in per se known manner by another group $X^{\ominus}$ or an alkyl group $R_1$ can be introduced.

3. Use of bis-(2,4-dinitrophenyl)-methyl-pyridinium compounds according to claim 1 as pH indicators.

4. Use according to claim 3 for the determination of ammonia in urea tests.

5. pH-determination agent, characterised by a content of compounds according to claim 1.

**Claim** for Contracting State AT

Process for the preparation of bis-(2,4-dinitrophenyl)-methyl-pyridinium compounds of the general formula I

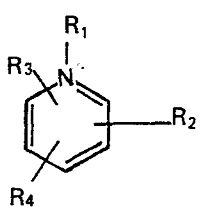

X (I)

wherein one of the groups $R_1$ and $R_2$ represents a bis-(2,4-dinitrophenyl)-methyl group and the other a lower alkyl radical, especially methyl or t.-butyl group, or, for the case in which $R_1$ represents the bis-(2,4-dinitrophenyl)-methyl group, $R_2$ signifies hydrogen atom or a trifluoromethyl group and $R_3$ and $R_4$ represent hydrogen or, together with the carbon atoms to which they are attached, a benzene ring, and $X^{\ominus}$ represents a suitable acid anion or the compound is present as betaine, characterised in that one
a) nitrates a compound of the formula I′

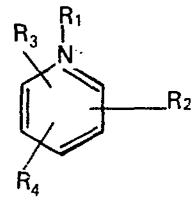

X (I′),

in which one of the groups $R_1$ and $R_2$ represents a diphenylmethyl group and the other groups have the same meaning as in formula I, or
b) for the case in which $R_1$ represents a bis-(2,4-dinitrophenyl)-methyl group, reacts a compound of the formula II

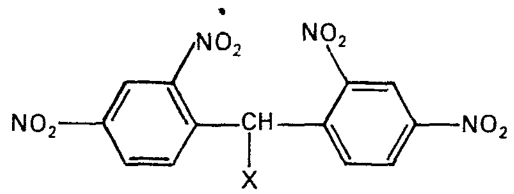

(II),

in which X represents a Cl, Br or I, with a compound of the formula III

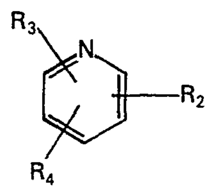

(III),

in which $R_2$ represents hydrogen or a lower alkyl group and $R_3$ and $R_4$ have the above meaning, whereafter the group $X^{\ominus}$ can be replaced in per se known manner by another group $X^{\ominus}$ or an alkyl group $R_1$ can be introduced.

**Revendications** pour les Etats contractants: BE, CH, DE, FR, GB, IT, LU, NL, SE

1. Dérivés du bis-(2,4-dinitrophényl)-méthyl-pyridinium de formule générale I:

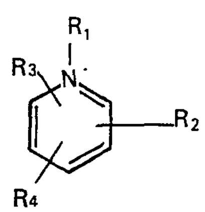

X (I)

dans laquelle un des groupes $R_1$ et $R_2$ est un groupe bis-(2,4-dinitrophényl)-méthyle et l'autre un groupe alkyle inférieur, en particulier un groupe méthyle ou tert.-butyle ou, dans le cas où $R_1$ est le groupe bis-(2,4-dinitrophényl)-méthyle, $R_2$ est de l'hydrogène ou un groupe trifluorométhyle,
$R_3$ et $R_4$ sont chacun de l'hydrogène ou forment ensemble avec l'atome de carbone auquel ils sont liés un noyau benzénique, et, $X^{\ominus}$ est un anion acide approprié, ou bien le dérivé se présente sous la forme d'une bétaïne.

2. Procédé pour la préparation de dérivés du bis-(2,4-dinitrophényl)-méthyl-pyridinium selon

la revendication 1, caractérisé en ce que
a) on soumet un composé de formule I'

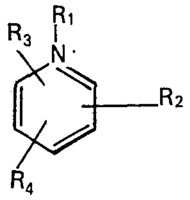

X (I'),

dans laquelle un des groupes $R_1$ et $R_2$ est un groupe diphénylméthyle et les autres groupes ont la même signification que dans la formule I, à une nitration, ou,
b) dans le cas où $R_1$ est le groupe bis-(2,4-dinitrophényl)-méthyle, ou fait réagir un composé de formule II

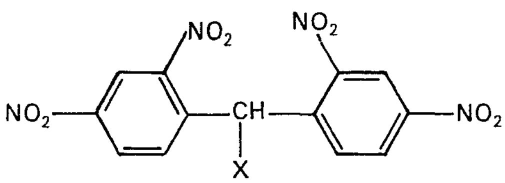

(II),

dans laquelle X est un atome de Cl, de Br ou de J, avec un composé de formule III

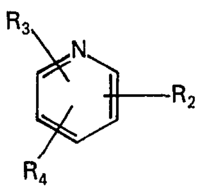

(III),

dans laquelle $R_2$ est de l'hydrogène ou un groupe alkyle inférieur et $R_3$ et $R_4$ ont la signification cidessus donnée, et après cette réaction, éventuellement on remplace de façon connue en soi le groupe $X^{\ominus}$ par un autre groupe $X^{\ominus}$, ou on introduit un groupe alkyle $R_1$.

3. Utilisation de dérivés du bis-(2,4-dinitrophényl)-méthyl-pyridinium selon la revendication 1 en tant qu'indicateurs de pH.

4. Utilisation selon la revendication 3 pour le dosage d'ammoniac dans des recherches d'urée.

5. Agents pour l'indication du pH caractérisés en ce qu'ils ont une teneur en dérivés selon la revendication 1.

**Revendication** pour l'Etat contractant AT

Procédé pour la préparation de dérivés du bis-(2,4-dinitrophényl)-méthyl-pyridinium de formule générale I

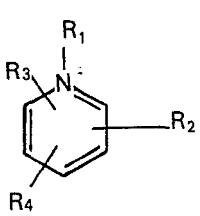

X (I)

dans laquelle un des groupes $R_1$ et $R_2$ est un groupe bis-(2,4-dinitrophényl)-méthyle et l'autre un groupe alkyle inférieur, en particulier un groupe méthyle ou tert.-butyle ou, dans le cas où $R_1$ est le groupe bis-(2,4-dinitrophényl)-méthyle, $R_2$ est de l'hydrogène ou un groupe trifluorométhyle,
$R_3$ et $R_4$ sont chacun de l'hydrogène ou forment ensemble avec l'atome de carbone auquel ils sont liés un noyau benzénique, et, $X^{\ominus}$ est un anion acide approprié, ou bien le dérivé se présente sous la forme d'une bétaïne, caractérisé en ce que
a) on soumet un composé de formule I'

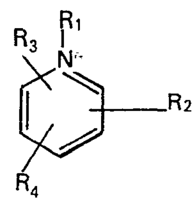

X (I'),

dans laquelle un des groupes $R_1$ et $R_2$ est un groupe diphénylméthyle et les autres groupes ont la même signification que dans la formule I, à une nitration, ou,
b) dans le cas où $R_1$ est le groupe bis-(2,4-dinitrophényl)-méthyle, ou fait réagir un composé de formule II

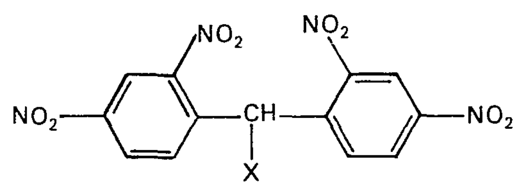

(II),

dans laquelle X est un atome de Cl, de Br ou de J, avec un composé de formule III

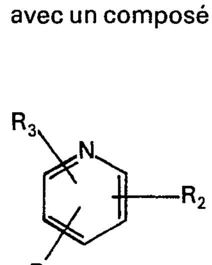

(III),

dans laquelle $R_2$ est de l'hydrogène ou un groupe alkyle inférieur et $R_3$ et $R_4$ ont la signification cidessus donnée, et après cette réaction, éventuellement on remplace de façon connue en soi le groupe $X^{\ominus}$ par un autre groupe $X^{\ominus}$, ou on introduit un groupe alkyle $R_1$.

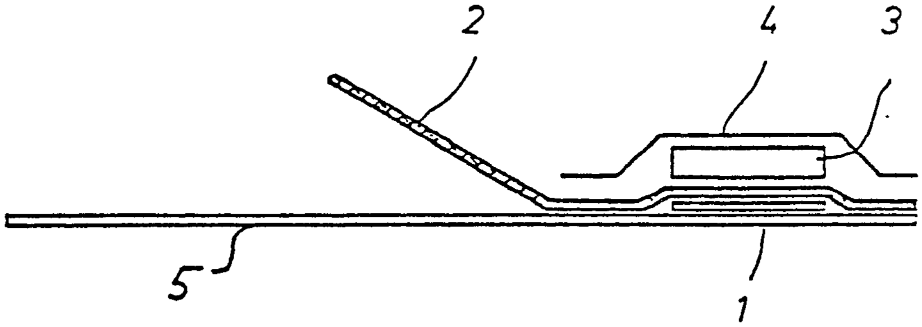


Fig. 1

# Fig. 2

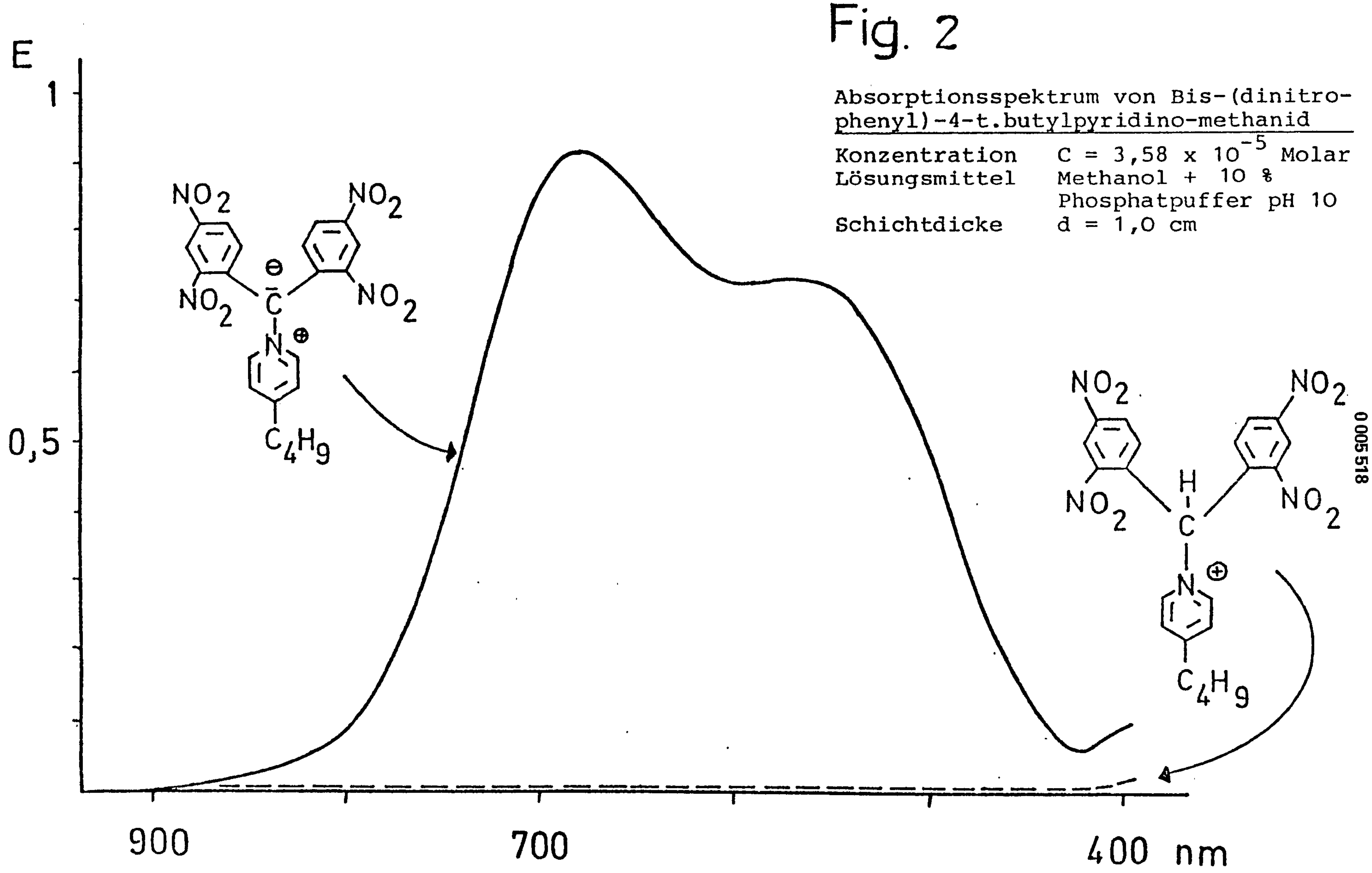
E
1
0,5
900
700
400 nm
Absorptionsspektrum von Bis-(dinitrophenyl)-4-t.butylpyridino-methanid
Konzentration C = 3,58 x 10^-5 Molar
Lösungsmittel Methanol + 10 % Phosphatpuffer pH 10
Schichtdicke d = 1,0 cm